# EUROPEAN PATENT APPLICATION

(11) **EP 2 551 670 A1**
(43) Date of publication of application: **30.01.2013**
(21) Application number: 11175584.9
(22) Date of filing: 27.07.2011
(51) Int. Cl.: G01N 33/50, G01N 33/542

(54) **NIK inhibitors cell-based assay**

(71) Applicant: Merck Patent GmbH, 64293 Darmstadt (DE)
(72) Inventor: The designation of the inventor has not yet been filed
(74) Representative: Merck Serono SA - Geneva Intellectual Property

(57) **Abstract**

The present invention relates to a cell-based assay for the screening and the identification of inhibitors of NIK protein. The method according to the present invention allows the identification of compounds that inhibit NIK activity related to the phosphorylation of IKK1. The assay combines the use of transfected Human Embryonic Kidney (HEK) cells with a non-radioactive, homogeneous proximity assay using an "acceptor" bead and a "donor" bead.

## Description

### Field of the invention

The present invention relates to a cell-based assay for the screening and the identification of inhibitors of NIK protein.

### Background of the invention

The nuclear factor- kappa B (NF-κB) family of transcription factors is comprised of a collection of structurally related proteins that participates in various biological processes, including immune responses, inflammation, cell death and survival (Thu Y.M. and Richmond A. Cytokine & Growth Factor Rev 2010, 21:213-226).

The mammalian NF-κB family comprises five members, including p65 (also known as ReIA), p50 (also known as NF-κB1), p52 (also known as NF-κB2), RelB and c-Rel, which form several dimeric complexes able to regulate transcription and expression of various downstream gene targets through the binding to the κB enhancer. The typical form of NF-κB consists of the p50/p65 heterodimer. Such a complex is normally sequestered in the cytoplasm in an inactive form as long as it is bound to the members of the inhibitor of κB proteins (IκBs), particularly IκBα (Sun S.C., Cell Res 2011, 21:71-85).

Two main pathways controlling the activation of NF-κB, namely the canonical and non-canonical pathways, are now known. The well known canonical NF-κB pathway is triggered by a wide range of extracellular stimuli including pro-inflammatory cytokines such as TNFα, IL-1β, IL-6 and CD40L. This pathway is typically mediated by the Inhibitor of κB Kinase trimeric complex Kinases (IKKα, -β and -γ), which phosphorylate IκB members, leading in turn to the ubiquitination and complete degradation of IκBs by the proteasome and allowing the released RelA/p50 heterodimers to translocate into the nucleus to initiate gene transcription (Hayden M.S. et al., Cell 2008, 132:344-362; Vallabhapurapu S. et al., Annu Rev Immunol 2009, 27:693-733).

In addition to the well-known canonical pathway, a non-canonical pathway also exists. This alternative NF-κB activation pathway is induced by a subset of tumor necrosis factor ligand (TNFL) family members such as CD40 ligand (CD40L), B-cell activating factor (BAFF) or ligands for the lymphotoxin β receptor (LTβR). Experimental evidences suggest that this pathway is important in regulating many biological processes such as secondary lymphoid organ development, B-cell survival and maturation and induction of specific chemokines involved in adaptive immunity (Dejardin E., Biochem Pharmacol 2006, 72:1161-1179).

This pathway involves the activation of ReIB/p52 complex by inducing the processing of the p100 precursor protein into the mature p52 by IKKα and the NF-kappa-B-inducing kinase (NIK) protein. Both kinases phosphorylate p100, which contain an IκB-like C-terminal portion and consequently function as NF-κB inhibitor, leading to a partial proteolysis of the p100 protein to obtain the active form p52. The processing of p100 thus serves to both generate p52 and induce the nuclear translocation of the ReIB/p52 heterodimer (Coope H.J. et al., EMBO J 2002, 15:5375-5385).

Using overexpression of a dominant negative mutant, NIK was first identified as a kinase that mediates the proximal signaling downstream of TNFα and IL-1 receptors, and the kinase activity of NIK is required for this process (Malinin N.L. et al., Nature 1997, 385:540-544). NIK also mediates stimulation through other receptors such as CD27, CD30, CD40, LTβR and BAFFR. When over-expressed, NIK leads to activation of NF-κB, therefore protecting cells from undergoing TNFα-induced apoptosis.

NIK has been demonstrated to interact with, and activate IKKα and IKKβ. However, NIK phosphorylates IKKα to a greater extent than IKKβ. Thus, NIK serves as an upstream IKK-complex kinase responsible for the proximal signaling of various cytokine receptors.

IKKα (also known as IKK1) is a preferential substrate for NIK. NIK directly phosphorylates IKKα on Ser-176, and mutation of this residue to alanine prevents NIK activation of IKKα, thus disrupting signals from IL-1 and TNFα stimuli (Ling L. et al., Proc Natl Acad Sci 1998, 95:3792-3797).

From the preferential interaction with IKKα, it can be deduced that NIK is a more important kinase for IKKα than for IKKβ and that NIK is only essential for the non-canonical pathway. However, depending on inducer and cell type, NIK does play a role in both the canonical and non-canonical NF-κB pathways. Data from studies using mouse embryonic fibroblasts (MEFs) with NIK knock-out or with a naturally occurring NIK defective mutant, alymphoplasia (aly/aly), support this notion (Shinkura R. et al., Nature Genetics 1999, 22:74-77).

NIK is required for the processing of p100 to p52, thus confirming its role in the non-canonical pathway (Xiao G. et al., Mol Cell 2001 7:401-409). The kinase activity of NIK or IKKα is not required for the recruitment of p100 to both kinases, however, it is required for ligand-induced proteolysis to occur (Xiao G. et al., J Biol Chem 2004, 279:30099-30105). Surprisingly, NIK is not required for the constitutive processing of p100 to p52. The mechanism by which NIK controls p100 to p52 processing is through its stability. Upon stimulation with ligands such as CD40L or BAFF, basally translated NIK is stabilized to induce p100 to p52 processing (Qing G. et al., J Biol Chem 2005, 280(49):40578-82).

In summary, NIK is a kinase that functions in the canonical pathway, but is required only for NF-κB activation in response to certain ligands. The physiological significance of this selective requirement remains elusive. Perhaps, NIK lies at a cross road of the canonical and non-canonical pathways of NF-κB, maintaining the positive and negative regulatory balance between these two pathways. For example, NIK activates the canonical pathway, and thus possibly induces the production of p100, a fourth IκB of the NF-κB family (negative feedback mechanism). At the same time, NIK is the kinase responsible for processing this IκB so that it will liberate NF-κB heterodimers such as ReIA/p50 to activate gene transcription (positive feedback mechanism). It therefore has become exceedingly clear that NIK plays a dynamic role in NF-κB activation, coordinating the intricate balance between the canonical and non-canonical regulations of NF-κB.

Because of the important role that NIK could have in regulating biological processes related to many diseases, and in inflammatory/autoimmune diseases in particular, a series of biochemical and cell-based assay for NIK have been reported.

Ling L. et al. (Proc Natl Acad Sci 1998, 95:3792-3797) discloses the use of HEK cells transiently transfected with expression plasmids encoding FLAG-NIK-wt and FLAG-IKK-α (kinase-dead). After a 36 hours transfection, immunopurified proteins were incubated with γ-[³²P]-ATP, resolved by SDS-PAGE, and analyzed by autoradiography. These studies showed that FLAG-NIK-wt specific phosphorylation of FLAG-IKK-α(kinase-dead) occurred. Further experiments were performed using FLAG-IKK-α(kd)-S176A, FLAG-IKK-α(kd)-T179A and FLAG-IKK-α(kd)-S180A as substrates and these showed that the major NIK-mediated phosphorylation site is Ser-176 of the IKK-α(kd). This assay has the potential to detect all phosphorylation events of FLAG-IKK-α(kd) and therefore it is unclear to what extent the specific phosphorylation of Ser-176 of the FLAG-IKK-α(kd) has taken place during the 36 hours transfection when protein expression and additional signaling events would have occurred, nor if this approach is amenable to high-throughput inhibitor discovery.

US 6,841,556 discloses the ability of a series of pyrazoloisoquinoline derivatives to inhibit NIK in various *in vitro* assay systems. However, no details of the *in vitro* NIK assays are provided. WO/2009/158011 discloses a series of *in vitro* NIK autophosphorylation assays based on chemiluminescence (CL) and homogenous time resolved fluorescence (HTRF) formats. Both formats utilized a truncated form of NIK containing only the catalytic domain with substantially increased autophosphorylation activity compared to the full-length protein. The NIK proteins were expressed in a baculovirus expression system, purified and biotinylated. For the CL assay, 50 nM of biotinylated-NIK was incubated with compounds and 50 µM ATP in a buffer containing 20 mM Tris-HCI pH 7.5, 40 mM MgCl₂, and 1.5 mM DTT at room temperature for 1 hour. The reaction mixtures are then immobilised on a streptavidin-coated plate and the autophosphorylation activity of NIK detected with an anti-phospho-serine/threonine antibody and a horseradish peroxidase-conjugated secondary antibody.

For the HTRF assay, 10 nM of NIK was incubated with compounds in the presence of 20, 100, 200, or 500 µM of ATP in a buffer containing 20 mM of HEPES pH 7.5, 20 mM MgCl₂, 0.2% Tween 20 and 1 mM DTT at room temperature for 2 hours. The reactions were stopped by adding 5 mM of EDTA, 12.5 ng/ml of Eu-labelled anti-phospho-serine/threonine antibody and 1 nM streptavidin-Alexa647. After a 2 hours incubation, the fluorescence was measured at 615 nm and 665 nm, while the specific enzymatic activity was calculated by the signal ratio between 665 nm and 615 nm. As these assays make use of truncated NIK protein, inhibitor screening would only detect molecules that prevent this event.

Mortier, J. et al. (Bioorg & Med Chem Lett 2010, 20:4515-4520) discloses the use of an *in vitro* NIK assay using the ProQinase ³³PanQinase technology. NIK was expressed in Sf9 insect cells as a human recombinant GST-fusion protein and was purified by affinity chromatography using GSH-agarose. The purity of NIK was checked by SDS-PAGE/silver staining and the identity verified by mass spectroscopy. The NIK activity was measured by the incorporation of ³³P into the artificial substrate RBER-CHKtide, an artificial fusion protein expressed in *E. coli* consisting of an N-terminal GST-tag separated by a thrombin cleavage site from a fragment of the human retinoblastoma protein RB1 consisting of amino acids S773-K928, followed by 11 Arg residues (ER) and a peptide sequence KKKVRSGLYRSPSMPENLNRPR (CHKtide). As this assay makes use of an artificial substrate, it is not clear whether its use in inhibitor screening would allow detect molecules that would be physiologically relevant.

Hassan, N.J. et al. (Biochem J 2009, 419:65-73) discloses the use of baculovirus-infected Sf9 insect cells to monitor FLAG-NIK-wt mediated phosphorylation of Ser-176/Ser-180 of its downstream substrate FLAG-IKKα-(kd). This assay was in a time-resolved fluorescence resonance energy transfer (TR-FRET) format in a 384 well microtitre plates that utilized a specific antibody against the Ser-176/Ser-180 of IKKα. This assay was used for screening purposes against a kinase focussed small molecule library. A number of apparent NIK inhibitors were identified from the screening campaign and a selection thereof were further analysed, one of which was shown to be non-cytotoxic, interacted directly with NIK in a fluorescence polarisation assay and inhibited lymphotoxin-induced p52 translocation to the nucleus. Although this provided strong support that the insect cell system was able to identify *bona fide* NIK inhibitors, the cell background was not human.

There is a need for a robust and physiologically relevant assay to identify NIK inhibitors. It is desirable that such an assay can be easily and rapidly carried out using currently available analytical instrumentation. Approaches so far have been unable to produce full-length NIK enzyme with *in vitro* activity using IKKα as a substrate. Although truncated forms of NIK can be generated in insect cells which undergo autophosphorylation, these variants do not phosphorylate IKKα. Therefore it is uncertain that inhibitors in this assay have relevance in *vivo.*

This lack of robust, NIK-specific assays is a major cause for the paucity of known inhibitors against this potentially important disease target; only a few series of such compounds are reported so far (Mortier J. et al., Bioorg & Med Chem Lett 2010, 20:4515-4520; WO/2009/158011). For instance, the first claimed NIK inhibitors are now known to affect targets elsewhere in the NF-κB signaling cascade, suggesting that these compounds were discovered using a cell-based reporter assay.

### Summary of the invention

It is an object of the present invention to provide a cell-based assay useful for the screening and the identification of NIK inhibitors, i.e. candidate molecules inhibiting NIK activity.

The method according to the present invention allows the identification of compounds that inhibit NIK activity related to the phosphorylation of IKK1. The assay combines the use of transfected Human Embryonic Kidney (HEK) cells with a non-radioactive, homogeneous proximity assay using an "acceptor" bead and a "donor" bead. HEK cells are transfected with at least one expression vector containing a DNA encoding the full length human NIK protein and a DNA encoding a mutated form of IKK1. The mutated IKK1 protein substrate contains a K44A mutation, also named KD (Kinase Dead) mutation. The K44A mutation renders IKK1, the full-length NIK natural substrate protein, incapable of autophosphorylation at the Ser-176 residue (Nakano H. et al., Proc Natl Acad Sci 1998, 95:3537-3542). The transfected HEK cells are cultured in conditions which allow the production of NIK and the mutated IKK1 proteins. Since NIK specifically phosphorylates IKK1 at the Ser-176 residue, the use of the IKK1-KD mutated protein as a substrate results in only NIK-mediated phosphorylation of the substrate. Inhibition of IKK1-KD phosphorylation allows therefore the identification of compounds having an inhibitory effect on NIK.

In the assay according to the present invention the transfected HEK cells containing NIK and IKK1-KD are cultured in well plates. The cell culture is preferably carried out a temperature close to 37°C for a suitable time period in the presence of 5% CO₂. The temperature is selected so as to facilitate penetration of the compound to be tested (candidate molecule) into the transfected HEK cells, as well as to maintain viable HEK cells. The ability of a candidate molecule to inhibit NIK-mediated phosphorylation of IKK1-KD is then detected through the use of a bead based luminescent proximity system.

In a bead based luminescent proximity system a donor and an acceptor pair of reagent-coated (micro) beads are brought into proximity by a biomolecular interaction of binding partners immobilized to these beads.

Proximity of acceptor to donor beads relies upon on the biological interaction of the molecules bound to them. The most common assay is constructed by capturing one binding partner onto the donor beads and the other partner onto the acceptor beads. When the partners are brought to proximity by interacting with the same substrate, electromagnetic energy is transferred from a donor to acceptor beads upon laser excitation and a signal is produced. The signal is then detected through any suitable means, such as the Envision 2103 Multilabel Reader. The advantages of such a technology are a very high sensitivity, a very low signal background, high signal/background ratios, miniaturization and cost effectiveness.

The assay of the present invention allows a simple, sensitive and rapid way to achieve screening of candidate molecules to identify NIK inhibitors against IKK1 as a substrate. In the context of the present invention, the terms "assay" and "method" are used interchangeably.

### Abbreviations

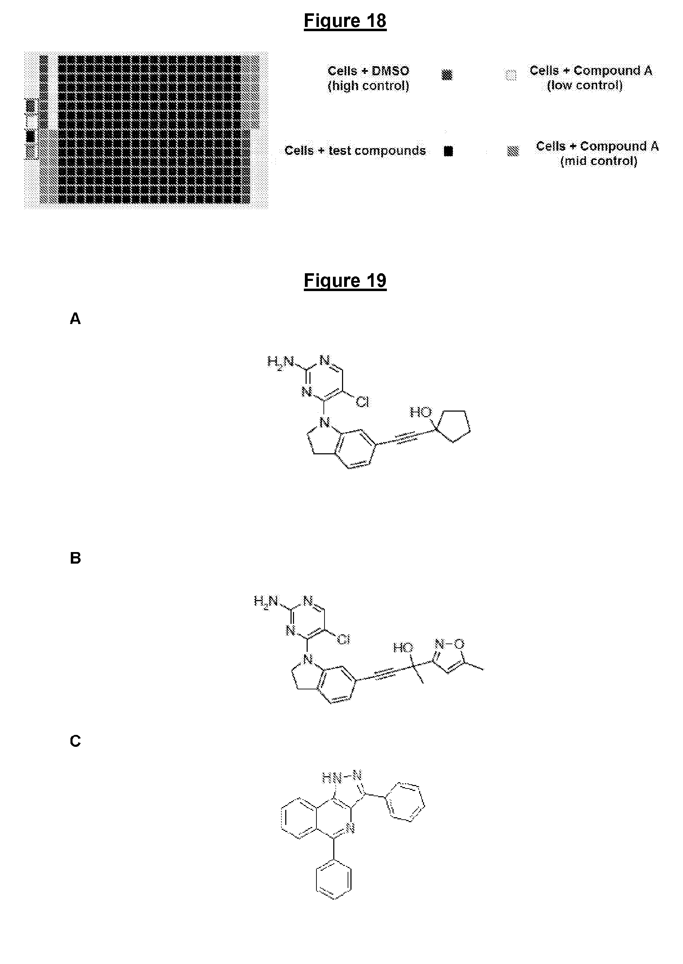

- HEK: Human Embryonic Kidney
- Sf9 cells: *Spodoptera frugiperda* cells
- fl: Full length
- KD/kd: kinase dead
- K44A: Lys-44-Ala mutant (kinase dead)
- NIK: NF-κB inducing kinase
- NIK-fl: full length NF-κB inducing kinase
- IKK-α: Inhibitor of NF-κB Kinase subunit alpha
- IKK-β: Inhibitor of NF-κB Kinase subunit beta
- IKK1-KD: kinase dead full length IKK-α
- FLAG-IKKα (kd): kinase dead FLAG tagged IKK-α
- FLAG-IKKα (kd)-S176A: kinase dead FLAG tagged IKKα Ser-176-Ala mutant
- FLAG-IKKα (kd)-T179A: kinase dead FLAG tagged IKKα Thr-179-Ala mutant
- FLAG-IKKα (kd)-S180A: kinase dead FLAG tagged IKKα Ser-180-Ala mutant
- hNIK1-fl-wt-pFlagN: wild type FLAG tagged full length human NIK plasmid
- hNIK1-fl-wt-FlagN: wild type full length FLAG tagged human NF-κB inducing kinase
- IKK1-KD-pFlagN: kinase dead FLAG tagged IKK-1 plasmid
- IKK1-KD-FlagN: kinase dead FLAG tagged IKK-α
- AlphaScreen: Amplified Luminescent Proximity Homogeneous Assay
- AlphaScreen SureFire IKKα (phospho-Ser-176/180) Assay Kit: AlphaScreen kit to quantify Ser-176/180 phosphorylated IKKα protein in cells
- Compound A relates to the molecule depicted in Figure 19 (A)
- Compound B relates to the molecule depicted in Figure 19 (B)
- Compound C relates to the molecule depicted in Figure 19 (C)

### Description of the figures

**Figure 1****.** (**A**) AlphaScreen SureFire IKKα (phospho-Ser176/180) readout after 24 hr for single, dual and non transfected HEK cells (hNIK1-fl-wt-pFlagN and IKK1-KD-pFlagN plasmids); (**B**) As for (**A**) but a 48 hr transfection. The AlphaScreen signal was only observed when HEK cells were dual transfected with hNIK1-fl-wt-pFlagN and IKK1-KD-pFlagN plasmids.
**Figure 2****.** Immunocytochemistry of HEK cell dual transfected with hNIK1-fl-wt-pFlagN and IKK1-KD-pFlagN plasmids.
**Figure 3****.** (**A**) AlphaScreen signal for phosphorylated-IKK1-KD-FlagN versus total DNA for JetPEI dual transfected HEK cells with hNIK1-fl-wt-pFlagN and IKK1-KD-pFlagN plasmids for 24 hrs (**B**) Dose-responses for Compound A at 0.0625 - 10 µg/ml total DNA for JetPEI dual transfected HEK cells with hNIK1-fl-wt-pFlagN and IKK1-KD-pFlagN plasmids for 24 hrs.
**Figure 4****.** Dose-response curves for compounds using dual jetPEI hNIK1-fl-wt-pFlagN and IKK1-KD-pFlagN transfected HEK cells (**A**) Experiment 1: AlphaScreen signal for phosphorylated-IKK1-KD-FlagN at 24 hrs; **(B)** Experiment 1: AlphaScreen signal for phosphorylated-IKK1-KD-FlagN at 48 hours; **(C)** Experiment 2: AlphaScreen signal for phosphorylated-IKK1-KD-FlagN at 24 hrs; **(D)** Cell viability studies using CellTiter-Glo assay.
**Figure 5****.** DMSO tolerance of transfected HEK cells.
**Figure 6****.** AlphaScreen signal stability assessment at 3 and 16 hour post addition of AlphaScreen detection reagents.
**Figure 7****.** (**A**) Typical performance of the mid, low and high controls in the HEK cell based assay in 384 well microtitre plates; (**B**) Statistics for 352 DMSO wells of a 384 well microtitre plate.
**Figure 8****.** (**A**) Z' for the 14 microtitre plates from the n=1 and n=2 HEK cell based assay study. Z' >0.5 for 9 plates and Z' >0.4 for 10 microtitre plates; (**B**) raw data for microtitre plate 4; **(C)** raw data for microtitre plate 5. Columns 1 and 2 and columns 23 and 24 are control columns; columns 3 to 22 are columns used for test compounds.
**Figure 9****.** Correlation of the duplicate screening data sets (n=1 and n=2) for the HEK cell based assay. The high, low and mid control populations are located in the expected positions. A square: 7 compounds showing >50% inhibition in n=2 data set only; B square: 13 compounds showing >50% inhibition in both n=1 and n=2 data sets; C square: 1 compound showing >50% inhibition in n=1 data set only.
**Figure 10****.** Visualisation of the 2,240 test compound screen output from the HEK cell based assay. Plate Id (X-axis), inhibition (n=1) (Y-axis) and inhibition (n=2) (Z-axis).
**Figure 11****.** Calculation of the hit rates of the HEK cell based assay at different % inhibition cutoff values.
**Figure 12****.** The output from the CellTiter-Glo assay of the 21 hits identified from the HEK cell based assay.
**Figure 13****.** (**A**) Correlation of the 21 hits from the HEK cell based assay and the output from the CellTiter-Glo assay. Of the 21 compounds, 5 gave a >50% inhibition window between the HEK cell based and CellTiter-Glo assay; (**B**) Summary of the results from Figures 8-12.
**Figure 14****.** Structures of the 9 compounds that exhibited a profile of NIK inhibition in the HEK cell based assay >50% and cytotoxicity <50%.
**Figure 15****.** Z' for the 7 microtitre plates from the n=3 HEK cell based assay in (A) table format and (**B**) graphical format.
**Figure 16****.** (**A**) Correlation of the n=2 and n=3 screening output for the HEK cell based assay; (**B**) Summary of the results (n=1, n=2 and n=3) relating to the 21 hits in the HEK cell assay and the CellTiter-Glo cell viability assay.
**Figure 17****.** (**A**)-(**G**) Analysis of all 21 assay microtitre plates (n=1, n=2 and n=3) from the HEK cell based assay by way of Z', location of hits on assay plates (compounds yielding >50% Inhibition on at least one test occasion) and reproducibility of the hits.
**Figure 18****.** Layout of the test compounds, high, low and mid controls used in the screening assays.
**Figure 19****.** (**A**) and (**B**) Examples of compounds reported as NIK inhibitors (WO 2009/158011); (**C**) Compound reported as NIK inhibitor (US 6,841,556).

### Detailed description of the invention

The invention relates to a cell based assay with transfected HEK cells to identify compounds that inhibit NIK-mediated phosphorylation of IKK1-KD. The efficacy of a test compound in inhibiting NIK-mediated phosphorylation of IKK1-KD can be measured in a standard microtitre plate format. In a preferred embodiment, the assay can be carried out in a High Throughput Screening (HTS) configuration.

The term "NIK-mediated phosphorylation" refers to the ability of the NIK protein to phosphorylate a substrate. NIK phosphorylates IKK1 protein substrate specifically at the Ser-176 residue. In the context of the present invention, NIK substrate is IKK1 protein with a K44A mutation, also named IKK1-KD. The use of such mutated substrate renders IKK1 incapable of autophosphorylation at the Ser-176 residue.

A "HEK cell-based assay", when used in the present invention, refers to an assay system which uses Human Embryonic Kidney (HEK) cells containing NIK and its substrate IKK1-KD used in a method according to the invention, for analyzing the effect of a substance added to said HEK cells.

According to the present invention, "transfected HEK cells" refers to Human Embryonic Kidney cells containing a DNA encoding the full length human NIK protein and a DNA encoding the full length NIK substrate IKK1-KD protein. The assay of the present invention combines the use of transfected HEK cells with a non-radioactive, homogeneous proximity assay using an "acceptor" bead and a "donor" bead.

In a preferred embodiment, a DNA encoding the full length human NIK protein and a DNA encoding the IKK1-KD protein are inserted into HEK cells by transfecting said HEK cells with a first expression vector containing a DNA encoding the full length human NIK protein and a second expression vector containing a DNA encoding the full length NIK substrate IKK1-KD protein.

In an alternative embodiment, the HEK cells can be transfected with a single expression vector containing a DNA encoding the full length human NIK protein and a DNA encoding the full-length NIK substrate IKK1-KD protein.

In order to transfect HEK cells, any suitable method known in the art can be used. In a preferred embodiment, the jetPEI transfection reagent is used.

Transfection of HEK cells can be transient or stable. In one embodiment, the transfection is transient.

AlphaScreen is a suitable example of a non-radioactive, homogeneous proximity assay using an "acceptor" and a "donor" bead. The acronym ALPHA stands for Amplified Luminescent Proximity Homogeneous Assay. AlphaScreen is a bead-based detection system used to study biomolecular interactions in a microplate format. Binding of molecules captured on the beads leads to an energy transfer from one bead to the other, ultimately producing a luminescent/fluorescent signal. Every AlphaScreen assay contains two bead types, donor beads and acceptor beads. Both bead types are coated with a hydrogel which minimizes nonspecific binding and self-aggregation, and provides reactive aldehyde groups for conjugating biomolecules to the bead surface.

The use of transfected HEK cells in the method of the present invention is more physiologically relevant compared to current assays used to identify candidate compounds which inhibit NIK-mediated phosphorylation activity as it uses a human cell background.

To the extent the addition of a FLAG sequence does not interfere with the activity of NIK protein or of the IKK1-KD protein to act as a NIK substrate, the DNA encoding for NIK and/or for IKK1-KD may contain an additional N-terminal FLAG tag.

The transfected HEK cells according to the present invention are cultured in any suitable medium for an appropriate time period. Exemplary culture medium is DMEM with 10% FBS. Transfected HEK cells are cultured at 5% CO₂ and 37°C +/- 2°C, which is a temperature that allows the best conditions for the compounds to be tested to penetrate the cells and, if active, inhibit NIK activity as well as to maintain viable cells.

The assay is preferably performed in a microtiter plate format so that multiple test compounds or the same test compound at various concentrations can be evaluated simultaneously. In a preferred embodiment, a 384 well microtitre plate is used. In an alternative embodiment, a 1536 well microtitre plate can be used.

The compounds to be tested are dissolved in a suitable solvent, such as dimethyl sulfoxide (DMSO) and added to the culture medium where transfected HEK cells are being cultured. The cells are then incubated with the test compounds at 37°C +/- 2°C and 5% CO₂ preferably for 24 hours. It can be appreciated by one skilled in the art that conditions of incubation of the test compounds with transfected HEK cells set in the assay of the present invention facilitate the passive diffusion through cell membranes of the compounds to be tested. Such diffusion varies dramatically over a range of 20-37°C, with the maximum diffusion being at 37°C (Garrick R.A. et al., Am J Physiol 1982, 243:C285-C292). When active transport is involved, the dependence of diffusion upon temperature is even more dramatic. Such culture conditions represent a remarkable improvement over previous screening methods for identification of NIK inhibitors, in particular over insect Sf9 cell based assays which are generally carried out at 22-25°C.

In one embodiment, in addition to transfected HEK cells cultured with test compounds, wells containing only transfected HEK cells without test compounds are included in the microtitre assay plate. Such wells provide for a negative control which can be used to determine the inhibition of NIK activity upon exposure of the cells to the test compounds.

In a further embodiment, additional wells containing only transfected HEK cells cultured in presence of a compound known to inhibit NIK-mediated phosphorylation of a substrate are included in the microtitre assay plate. Wells containing the transfected HEK cells together with compound known to be a NIK inhibitor provide for a positive control for the inhibition of NIK activity.

Inhibition or reduction of NIK activity upon exposure of transfected HEK cells to the test compound as compared to the negative control is indicative of the test compound being a NIK inhibitor. Test compounds which reduce NIK activity to the levels of the positive control are expected to be effective NIK inhibitors.

After a suitable time period of incubation of the test compounds with the culture of transfected HEK cells, the cell culture medium is removed from the wells and the transfected HEK cells are lysed. To lyse the transfected HEK cells, a lysis buffer is added to the wells containing transfected HEK cells, thereby obtaining a cell lysate from transfected HEK cells.

In order to identify candidate compounds able to inhibit NIK-mediated phosphorylation of IKK1-KD protein, in accordance to the present invention a bead-based luminescent proximity detection system, such as the AlphaScreen system, is used.

AlphaScreen is a bead-based, non-radioactive amplified luminescent proximity homogeneous assay used to study biomolecular interactions in a microtitre plate format. To understand how signal is produced, one must begin with an understanding of the beads. Every AlphaScreen assay contains two bead types, donor beads and acceptor beads. Each bead type contains a different combination of chemicals, which are key elements of the AlphaScreen technology. Donor beads contain a photosensitizer, phthalocyanine, which converts ambient oxygen to an excited form of O₂, singlet oxygen (¹O₂), upon laser illumination at 680 nm. Like other excited molecules, singlet oxygen has a limited lifetime prior to falling back to ground state. Within its 4 µsec half-life, singlet oxygen can diffuse approximately 200 nm in solution. If an acceptor bead is within that proximity, energy is transferred from the singlet oxygen to thioxene derivatives within the acceptor bead, subsequently culminating in light production at 520-620 nm. In the absence of an acceptor bead, singlet oxygen falls to ground state and no signal is generated.

A first type of bead is called "acceptor bead". Acceptor bead is a latex-based bead of approximately 250 nm in diameter. Acceptor beads contain thioxene derivatives and fluorophores and are conjugated with molecules which can interact with a binding partner. The "binding partner" is a molecule, such as for instance an antibody which specifically binds to a protein, which brings into contact the acceptor bead and a second species. The molecule-conjugated acceptor bead and the binding partner are used to form a mix that is added to the sample to analyse.

According to the present invention, the binding partner of the acceptor bead is an anti-phospho-Ser-176/180 antibody. Such antibody recognizes IKK1-KD. In particular, the antibody binds to the phosphorylated form of Ser-176 residue of the IKK1-KD protein. Also according to the present invention, the molecule conjugated to the acceptor bead is Protein-A. Protein-A is able to specifically bind to the Fc portion of the anti-phospho-Ser-176/180 antibody. The Protein-A conjugated acceptor beads and the anti-phospho-Ser-176/180 antibody will consequently create an acceptor complex (hereinafter designated also as "first mix") when mixed together.

Also according to the present invention, a "first mix" is a mix composed of a Protein-A coated bead and an anti-phospho-Ser-176/180 antibody. In the present method, the first mix is added to and incubated for 2 hours at 24°C with the cell lysate obtained form transfected HEK cells cultured in presence of a candidate compound to be tested for a NIK inhibitor activity.

A second type of bead is called "donor bead". Also donor bead is a latex-based bead of approximately 250nm in diameter. Donor beads contain a photosensitizer, phthalocyanine, which converts ambient oxygen to an excited form of O₂, singlet oxygen, upon laser illumination at 680 nm. Donor beads are conjugated with several types of molecules to form a molecule-coated donor bead. Such a conjugation allows the interaction of the donor bead with a binding partner. Such a binding partner is a molecule which brings into contact the donor bead and a second species. The binding partner can be for instance an antibody which specifically recognize a second species and interact with a molecule-coated donor bead by specific interactions. The molecule-conjugated donor bead and the binding partner are used to form a mix that is added to the sample to analyse.

According to the present invention, the binding partner of the donor bead is a biotinylated antibody. Such biotinylated antibody specifically recognizes IKK1-KD. In particular, the biotinylated antibody specifically binds to the IKK1-KD protein in a protein portion which does not comprise the Ser-176 residue. Also according to the present invention, the molecule conjugated to the donor bead is streptavidin. Streptavidin is able to recognize and specifically interact with high affinity with the anti-IKK1-KD biotynilated antibody. The streptavidin-coated donor beads and the anti-IKK1-KD biotinylated antibody will consequently create a donor complex (hereinafter designated also as "second mix") when mixed together.

Also according to the present invention, a "second mix" is a mix composed of a streptavidin-coated donor bead and a biotinylated antibody which binds to the IKK1-KD protein. In the present method, the second mix is added to and incubated for 1 hour at 24°C with the lysate obtained form transfected HEK cells cultured in presence of a candidate compound to be tested for a NIK inhibitor activity, in which a first mixed has been previously added.

Accoding to the method of the present invention, the cell lysate obtained from transfected HEK cells, after incubation with the first mix and the second mix, is excited with a high-intensity laser at 680 nm which induces the formation of singlet oxygen at the surface of the streptavidin-coated donor bead, following conversion of ambient oxygen to a more excited singlet state by the phthalocyanine photosensitizer present in the donor bead. The donor bead generates about 60,000 singlet oxygen molecules, resulting in a greatly amplified signal.

If an acceptor bead is in proximity of a donor bead, energy is transferred from the singlet oxygen to thioxene derivatives within the acceptor bead, and light is produced in the 520-620 nm range. If an acceptor bead is not in close proximity to the donor bead, no energy is transferred, and therefore no signal is generated.

In the presence of a test compound unable to inhibit NIK-mediated phosphorylation of IKK1-KD protein, the transfected HEK cell lysate will contain the Ser-176 phosphorylated form of the IKK1-KD protein. Consequently, the anti-phospho-Ser-176/180 antibody present in the first mix will interact with IKK1-KD and the acceptor complex will be in close proximity to the donor complex, thus producing a detectable signal in the AlphaScreen detection system upon laser excitation of the cell lysate.

In the presence of a test compound able to inhibit or reduce NIK-mediated phosphorylation of IKK1-KD protein, the transfected HEK cell lysate will contain less of the Ser-176 phosphorylated form of the IKK1-KD protein. Consequently, less of the anti-phospho-Ser-176/180 antibody present in the first mix will interact with IKK1-KD and a reduced amount of the acceptor complex will be in close proximity to the donor complex, with the consequent reduction or absence of a detectable signal in the AlphaScreen detection system upon laser excitation of the cell lysate.

In order to analyse the robustness of the AlphaScreen analysis, it is useful to calculate the Z' value. The Z' value is a dimensionless parameter that allows for comparison and evaluation of the performance of different assay technologies that use different reagents, formats and instruments. The Z' value takes into account the standard deviations of two count populations, and hence gives a more useful estimate of assay signal window than do signal/background ratio alone. Typically, a Z' value greater than 0.5 is considered a good index of the robustness of the assay.

Once candidate molecules having apparent NIK inhibiting activity have been detected, it may be appropriate to let them undergo a cytotoxic "counterscreen" assay which could help differentiate molecules showing inhibition activity because of a cytotoxic effect, or because they interfere with the expression of the recombinant NIK and substrate proteins by other means (apparent inhibitors), from bona *fide* inhibitors. A suitable assay could be a CellTiter-Glo cell viability assay.

Thus provided herein are methods for identifying a compound that inhibits NIK activity, comprising:
(a) providing HEK cells containing a DNA encoding for NIK and a DNA encoding for IKK1-KD (hereafter "transfected HEK cells") in a culture medium;
(b) incubating said transfected HEK cells with and without a candidate molecule to be tested at a temperature of 37°C +/- 2°C in the presence of 5% CO₂ for 24 hours;
(c) removing the culture medium;
(d) lysing the cells;
(e) adding to the cell lysate an acceptor complex followed by a donor complex;
(f) exciting the cell lysate with a laser illumination and
(g) measuring the signal arising upon laser excitation of cell lysate using a bead-based luminescent proximity detection system,
wherein a reduction or the absence of the signal obtained from a cell lysate deriving from transfected HEK cells cultured in the presence of a test compound is indicative of the inhibition of NIK-mediated phosphorylation of IKK1-KD, and wherein the acceptor complex comprises a molecule-coated bead and a binding partner which binds to the IKK1-KD protein and the donor complex comprises a molecule-coated bead and a binding partner which binds to the IKK1-KD protein.

In an alternative embodiment, a reduction of the signal obtained from a cell lysate deriving from transfected HEK cells cultured in the presence of a test compound compared to the signal obtained from a cell lysate deriving from transfected HEK cells cultured in the absence of a test compound is indicative of a reduction of NIK-mediated phosphorylation of IKK1-KD.

In a preferred embodiment, the DNA encoding for NIK and the DNA encoding for IKK1-KD are inserted into HEK cells by transfecting said HEK cells with a first expression vector containing a DNA encoding for NIK and a second expression vector containing a DNA encoding for IKK1-KD.

In one embodiment, the molecule-coated bead of the acceptor complex is a Protein-A coated bead and the binding partner of the acceptor complex is an antibody which specifically binds to the phosphorylated form of Ser-176 residue of the IKK1-KD.

In one embodiment, the molecule-coated bead of the donor complex is a streptavidin-coated bead and the binding partner of the donor complex is a biotinylated antibody which binds to the IKK1-KD protein.

In one embodiment, the bead-based luminescent proximity detection system is AlphaScreen detection system.

In one embodiment, the method further comprises the step of testing those candidate molecules showing a NIK inhibitory activity in a cytotoxic assay, thus allowing to distinguish candidate molecules having a NIK inhibitor activity associated to a cytotoxic effect from candidate molecules having NIK inhibitor activity not associated to a cytotoxic effect.

### Examples

### Example 1: Reagent confirmation pilot studies

### Materials and Methods

HEK cells (HEK293A, Invitrogen R705-07) were either dual or single transfected with hNIK1-fl-WT-pFlagN and/or IKK1-KD-pFlagN plasmids (kindly provided by Prof. David Wallach Weizmann Institute, Israel) using jetPEI transfection reagent (PEQLAB Biotechnologie GmbH). Transfection controls included buffer and non-transfected controls. Approximately 1x10⁶ HEK cells were transfected for each condition (single transfection, dual transfection or non-transfected control) at 20µg total DNA in T75 flasks containing 10 ml media using the protocol from the supplier of jetPEI. Cells were then incubated for 24hr and 48hr at 37°C and 5% CO₂. Cells were washed, trypsinized and dispensed into 384 well microtitre plates (25µl, 5,000 cells/well). Detection of NIK-mediated phosphorylation of IKK1-KD protein was performed using the AlphaScreen SureFire IKKα (phospho-Ser-176/180) Assay Kit (PerkinElmer, catalogue no. TGRKAS10K) together with the AlphaScreen IgG Detection Kit (Protein-A) (PerkinElmer, catalogue no. 6760617M). After medium removal and lysis of HEK transfected cells, 5 µl/well acceptor mix (containing anti-phospho-Ser-176/180 antibodies and Protein-A-conjugated acceptor beads) was added and the plate incubated for 2 hr at room temperature. 2 µl/well donor mix (containing streptavidin coated-donor beads and biotinylated antibodies), was added and microtitre plates were incubated for 1 hr at room temperature, before reading (PerkinElmer, Envision 2103 Multilabel Reader). In an additional control, HEK cell lysates from two single transfected preparations (hNIK1-fl-WT-pFlagN or IKK1-KD-pFlagN plasmids) were mixed and then analysed as described above.

### Results

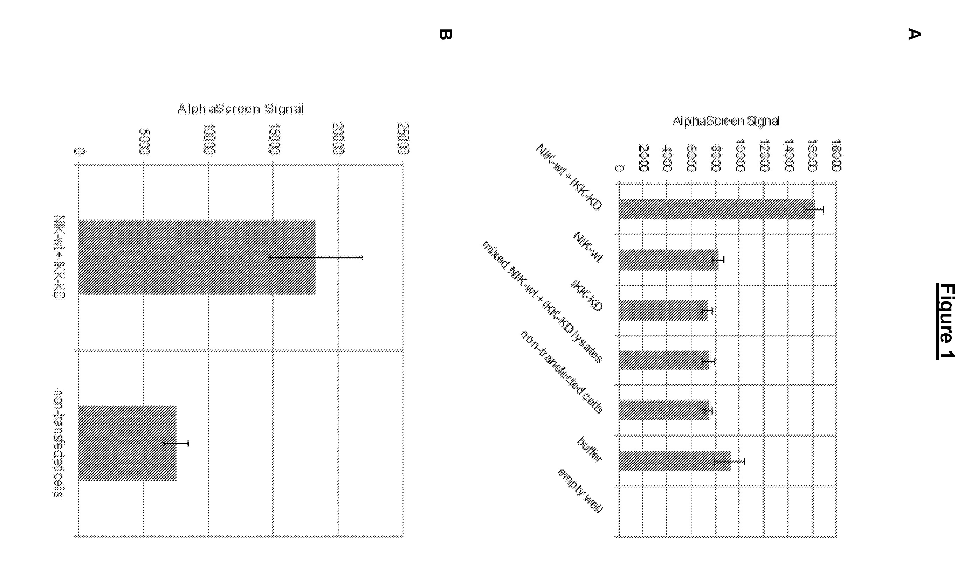

The results from the above experiments are shown in Figure 1.

The results clearly demonstrate that the IKK1-KD-FlagN undergoes hNIK1-fl-wt-FlagN mediated phosphorylation with both 24hr and 48hr transfections. In the absence of hNIK1-fl-wt-FlagN or IKK1-KD-FlagN proteins, no AlphaScreen signal was observed.

### Example 2: Confirmation of the presence of IKK1-KD-pFlagN by immunocytochemistry

Having demonstrated that the IKK1-KD-FlagN undergoes hNIK1-fl-wt-FlagN mediated phosphorylation, fluorescence imaging was performed to confirm the presence of both the NIK and IKK-1.

### Materials and Methods

HEK cells were dual transfected as described above. After 30 hr, the cells were fixed with acetone. Immunocytochemistry studies used an anti-NIK-T559 antibody (Santa Cruz, catalogue no. sc-12957) and an anti-IKKα-C-term antibody (Santa Cruz, catalogue no. sc-7121) and Alexa-488-secondary antibody (Invitrogen). Nuclei were stained with DAPI.

### Results

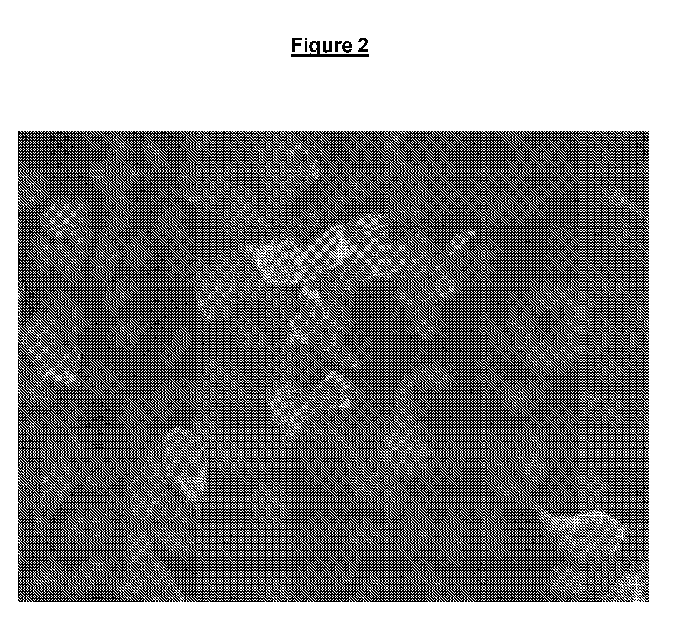

The results shown in Figure 2 demonstrate the presence of IKK1-KD-FlagN. Although these results were from a non-optimised experiment, they provided an early indication that a HEK cell based assay was feasible.

### Example 3: Confirmation of transfection efficiency with jetPEI

### Materials and Methods

HEK cells were dual transfected (with hNIK1-fl-WT-pFlagN and IKK1-KD-pFlagN plasmids, 1:1 ratio) over the range 0 - 10 µg/ml total DNA. The amount of jetPEI transfection reagent used was constant (2 µl/ml cell suspension). Upon transfection, cells were immediately dispensed into a 384 well microtitre plate (50µl/well containing 10,000 cells/well) with incubation at 37°C and 5% CO₂ for 24hr. Phosphorylated IKK1-KD-FlagN was detected using the AlphaScreen SureFire IKKα (phospho-Ser-176/180) readout as described above. A dose-response experiment with AlphaScreen SureFire readout was performed for Compound A, a molecule known to inhibit NIK.

### Results

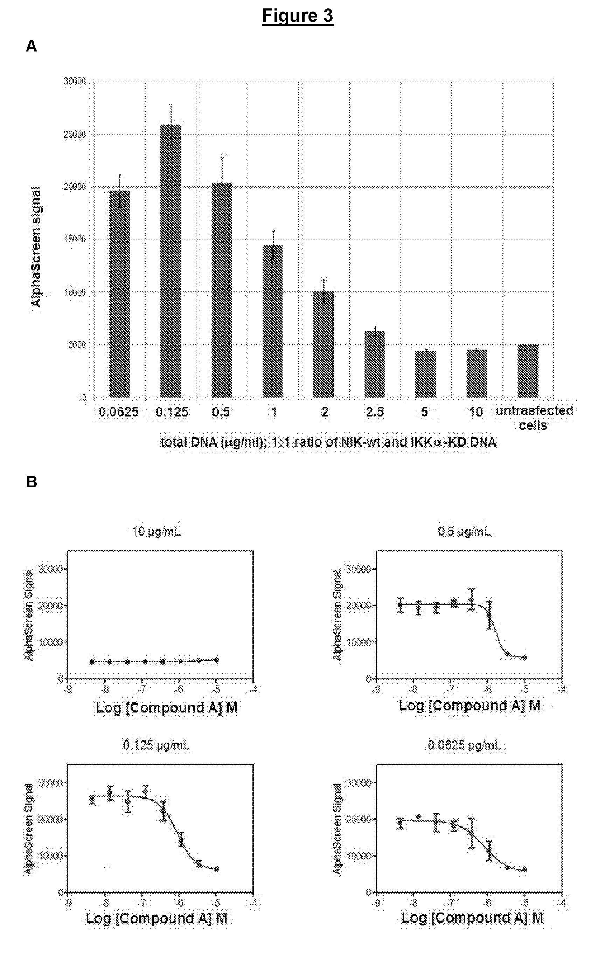

The results from the above experiments are shown in Figure 3.

Dual jetPEI transfected HEK cells result in the phosphorylation of IKK1-KD-FlagN, as detected by the AlphaScreen assay. As the DNA load is increased, a decrease in the AlphaScreen Signal is observed most likely due to inefficient transfection at high DNA concentrations as the amount of jetPEI was becoming limiting. Compound A provides an acceptable profile when the transfection is performed with a total DNA load of 0.125µg/ml.

### Example 4: Compound profiling with jetPEI transfected HEK cells

### Materials and Methods

In the first experiment, serial dilutions of four compounds were transferred to assay plates [Sunitinib, Compound A, Compound B and Compound C]. JetPEI dual transfected HEK cells (50 µl containing 10,000 cells/well) were dispensed into microtitre plates and incubated for 24hr and 48hr at 37°C and 5% CO₂. Plates were then processed and phosphorylated IKK1-KD-FlagN detected using the AlphaScreen SureFire IKKα (phospho-Ser-176/180) readout, as described in Example 1. A second experiment for Compound C, Compound A and Compound B was performed at the 24 hr time point only. For the same compounds, cell viability assays were performed using the CellTiter-Glo Luminescent Cell Viability Assay (Promega, catalogue no. G7570). Briefly, transfected HEK cells are cultured in the presence of candidate compounds to be tested for NIK inhibitor activity. After removing the medium, transfected HEK cells were lysed. 10 µl of CellTiter-Glo reagent provided in the assay kit was added in each well containing cell lysate and incubated at room temperature for 30 minutes. The plates were then read using the Envision 2103 Multilabel Reader. CellTiter-Glo assay permits the determination of the number of viable cells in culture based on quantitation of the ATP present, which indicates the presence of metabolically active cells.

### Results

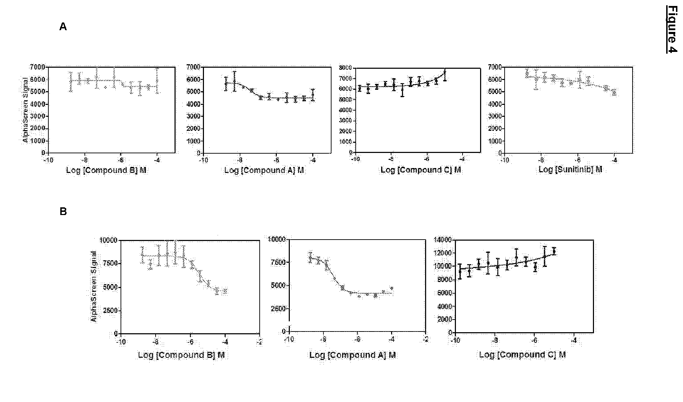

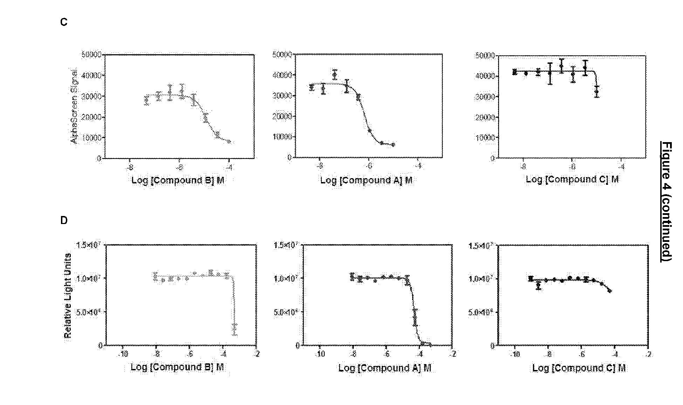

The results of the first experiment are shown in Figure 4(A) and (B). It is notable that Compound C was inactive in the assay whereas Compound A and Compound B appear as hNIK1-fl-wt-FlagN inhibitors. Sunitinib did not appear to inhibit the AlphaScreen Signal. These results were confirmed in experiment 2, see Figure 4(C). The dose-response curves for these compounds were well defined in terms of slope, minimum and maximum AlphaScreen signal. Compounds were also evaluated in a cell viability assay shown in Figure 4(D). No signal change was observed at concentrations of test compounds below 10 µM, suggesting that the effects shown in Figure 4(A), (B) and (C) are not due to compound cytotoxicity.

### Example 5: DMSO tolerance of jetPEI transfected HEK cells

The viability of HEK cells was investigated when exposed to DMSO with incubation at 37°C and 5% CO₂ for 24hr.

### Materials and Methods

Transfected HEK cells (50 µl containing 10,000 cells/well) was exposed to 0 - 10% v/v DMSO in a 384 well microtitre plate for a period of 24 hr at 37°C and 5% CO₂. CellTiter-Glo Luminescent Cell Viability Assay was performed as described in Example 4.

### Results

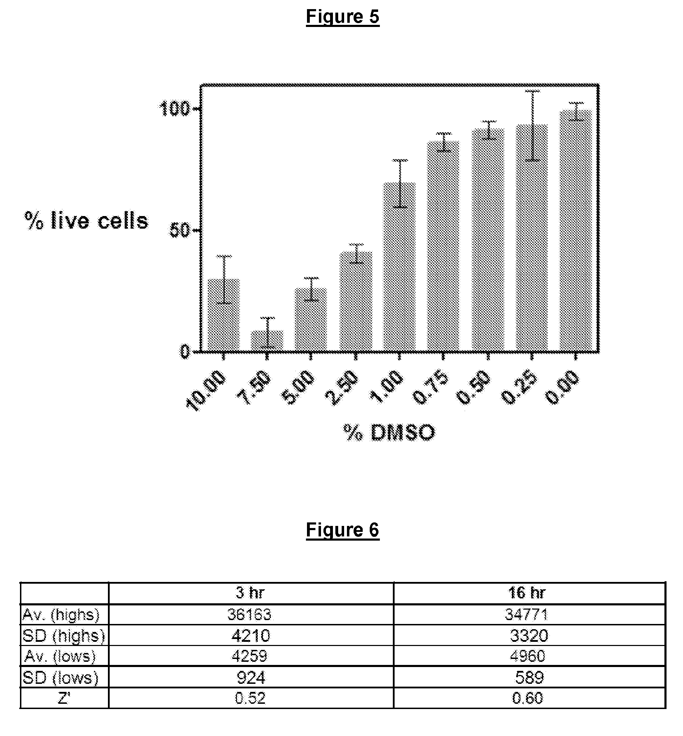

The results from the above experiments are shown in Figure 5.

The results shown in Figure 5 demonstrate that transfected HEK cells can tolerate exposure to DMSO up to 0.5% v/v for 24 hr with minimal loss of cell viability.

### Example 6: Z' and AlphaScreen Signal stability determination

The Z' and AlphaScreen Signal stability were determined.

### Materials and Methods

The HEK cell based assay was performed in 384 well microtitre plates. Each plate contained 4 control columns (64 wells) and 20 columns (320 wells) containing a total of 2,240 screened test compounds (ChemBridge Library) at 10 µM.

As a negative control (high control), 0.1% v/v DMSO was added to 32 wells. 10 µM of Compound A was added to 16 wells as a positive low control (100% inhibition) and 1 µM Compound A was added to 16 wells as a positive mid control (50% inhibition) using the plate layout depicted in Fig. 18.

Signal detection was obtained using an AlphaScreen SureFire readout as previously described after 3 and 16 hours.

### Results

The results from the above experiments are shown in Figure 6.

The Z' was >0.5 at both 3hr and 16hr after the addition of the AlphaScreen detection reagents.

### Example 7: DMSO and control compound study in microtitre plates

Before exposing the HEK cell based assay to 2,240 test compounds, assay performance was tested in microtitre plates containing DMSO wells along with mid, low and high controls.

### Materials and Methods

The assay protocol described in Example 6 was performed. The plate layout is shown in Fig. 18. Mid and low control wells contained 1 µM and 10 µM of Compound A, respectively. High control wells and 352 test wells contained 0.1 % v/v DMSO.

### Results

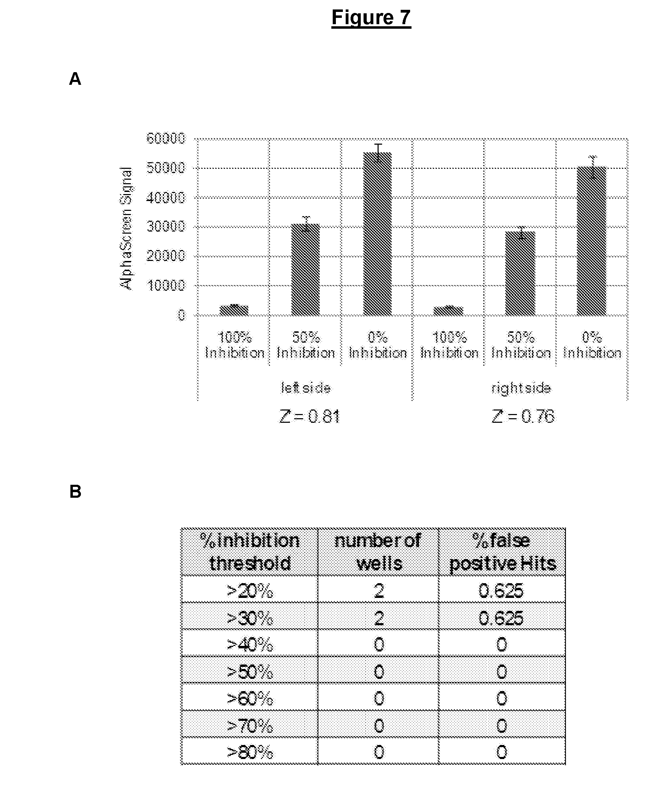

The results shown in Figure 7 (A) demonstrate that the HEK cell based assay performance was acceptable with Z' >0.5. Analysis of the DMSO wells of the microtitre shows a relatively low number of false positives arising from variations in liquid handling, see Figure 7 (B).

### Example 8: 2,240 test compound miniscreen (n=1 and n=2)

### Materials and Methods

The assay protocol as described in Example 4 was followed using the screening layout of Fig. 18. Fourteen 384 well microtitre plates were screened to provide a duplicate data set. Each microtitre plate contained 352 test compounds. The duplicate assays were performed on the same day but otherwise they were independent data sets that allow for assessing reproducibility of the assay.

### Results

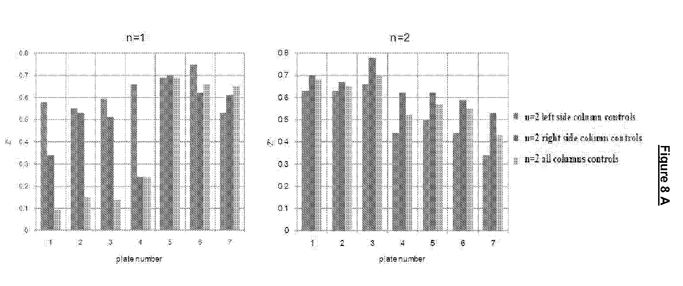

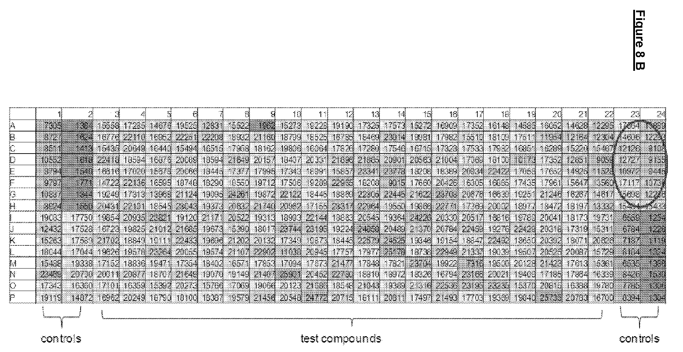

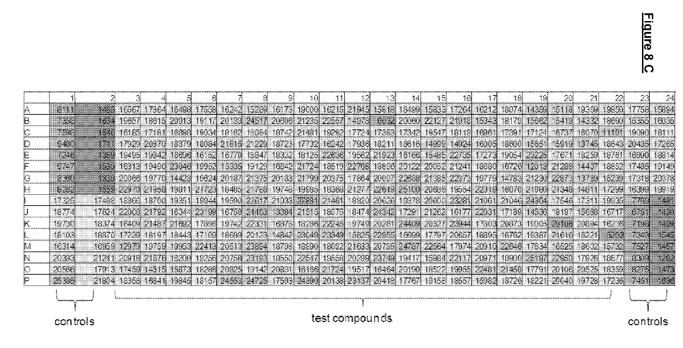

The results from the above experiments are shown in Figures 8-11.

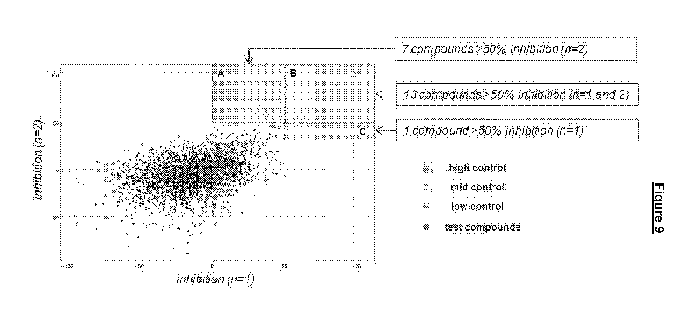

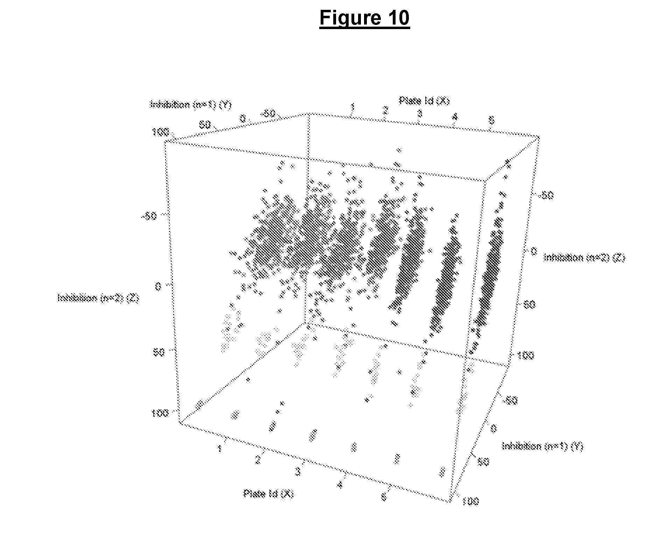

The results shown in Figure 9 show a good correlation between the n=1 and n=2 data sets. A higher variance in the high controls was observed than in the mid and low controls. The data from all plates was also visualised in a 3-D plot of plate (X-axis), inhibition (n=1) (Y-axis) and inhibition (n=2) (Z-axis), see Figure 10.

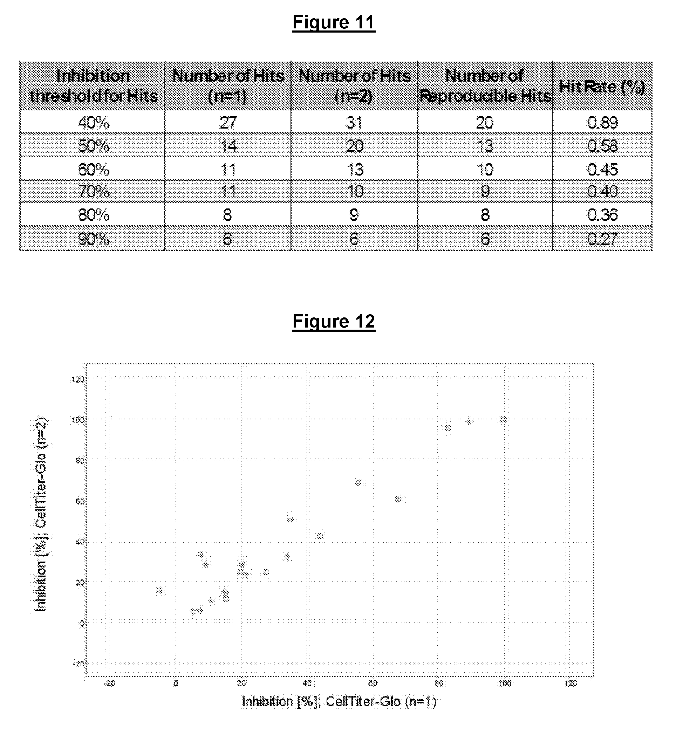

The results shown in Figure 11 indicate that increasing the percentage inhibition threshold increases the probability of hits being active on both test occasions. With a 50% inhibition threshold, 65% of the hits were active on both test occasions. With a 70% inhibition threshold, 81 % of the hits were active on both test occasions. With a 90% inhibition threshold, all of the hits were active on both n=1 and n=2 test occasions.

### Example 9: Evaluation of hit compounds in CellTiter-Glo assay

A screen was performed to identify hits arising from cytotoxic and assay interference effects. A total of 21 hits (compounds yielding >50% inhibition) were profiled in the CellTiter-Glo assay.

### Materials and Methods

The CellTiter-Glo Luminescent cell viability assay was performed as described in Example 4.

### Results

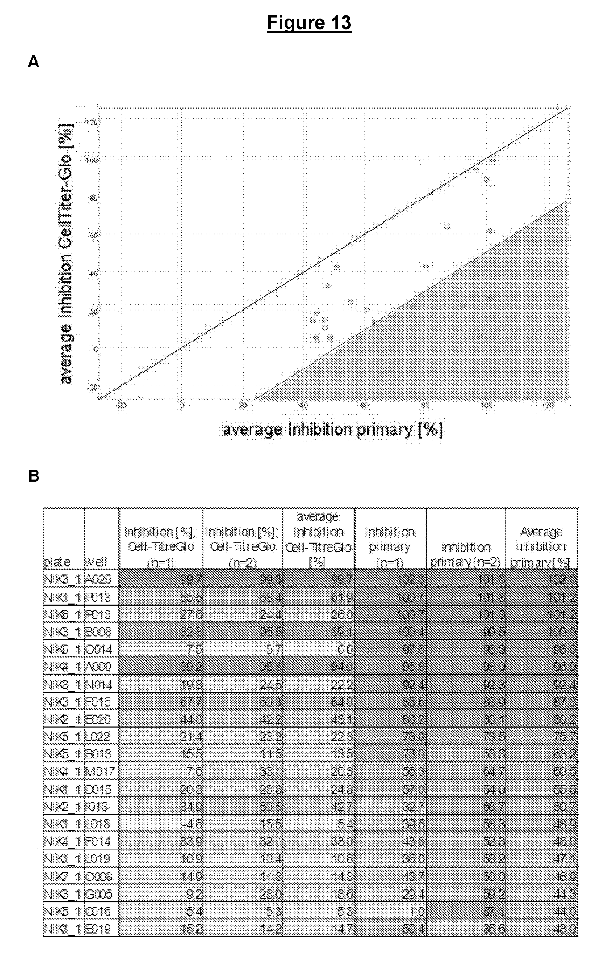

In the CellTiter-Glo assay, the 21 hit compounds exhibited a range of cytotoxicity profiles (see Figure 12). The correlation between cytotoxicity and the average of NIK inhibition in the HEK cell based assay results is shown in Figure 13 (A).

The results shown in Figure 13 (B) demonstrate that most of the 21 compounds that were hits in the HEK cell based assay produced a significant cytotoxic effect. Therefore these compounds are unlikely to be bona *fide* NIK inhibitors.

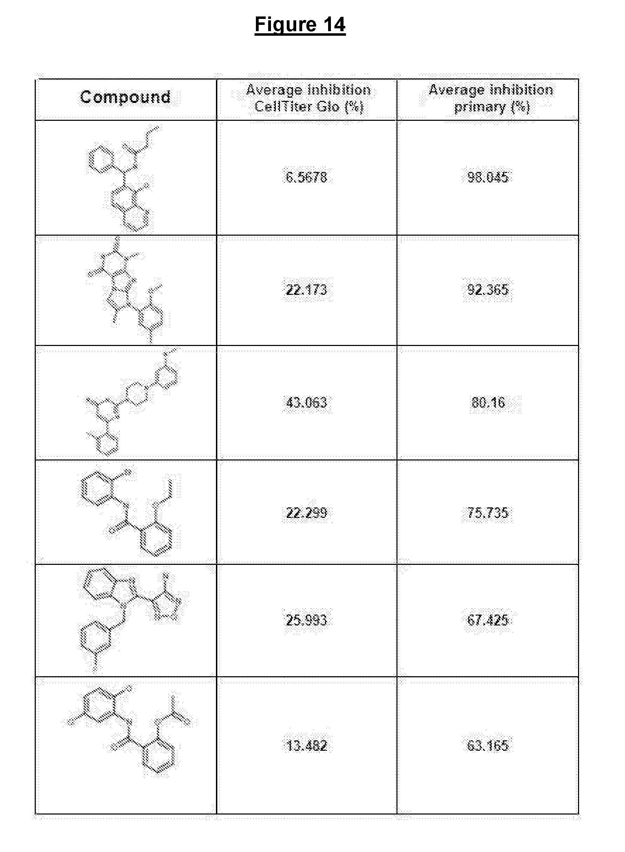

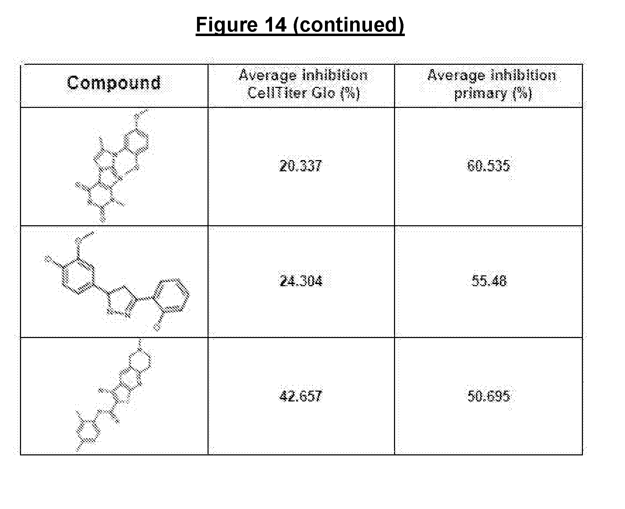

The structures of the 9 compounds that exhibited a profile with NIK Inhibition >50% and cytotoxicity <50% are shown in Figure 14.

In conclusion, of the 21 compounds that were evaluated, 9 compounds exhibited a profile with hNIK1-fl-wt-FlagN inhibition >50% and cytotoxicity <50%. The current HEK cell based assay is capable of detecting putative inhibitors of NIK activity (against IKK1-KD-FlagN substrate) that appear not to be exhibiting cytotoxicity.

### Example 10: HEK cell based assay in a 2,240 compound screen (n=3)

### Materials and Methods

The screen was repeated as described above.

### Results

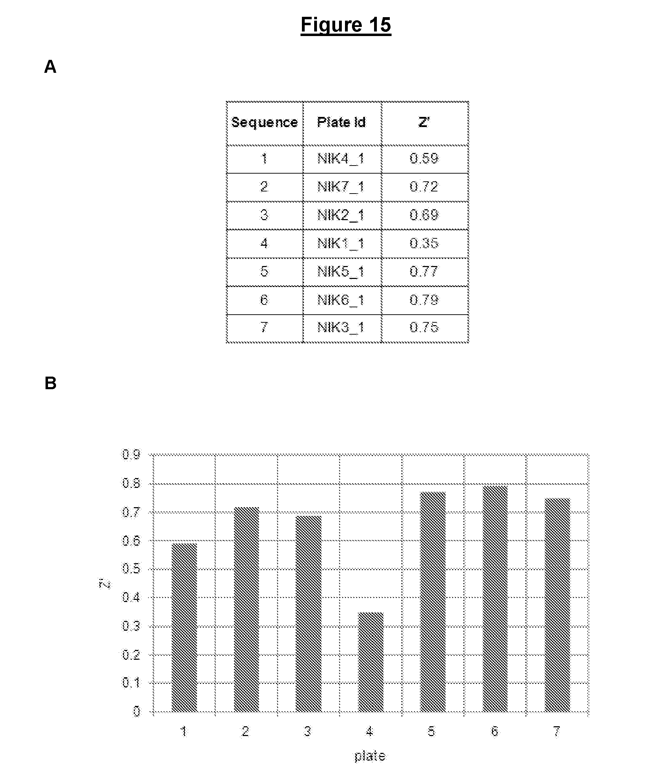

The results from the above experiments are shown in Figures 15-17.

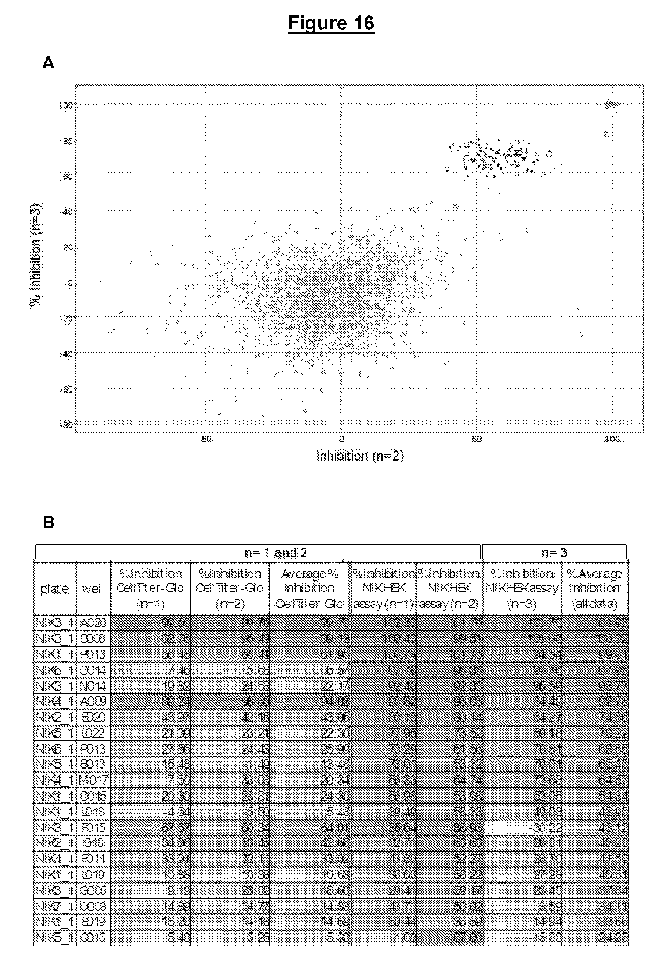

Of the 7 microtitre plates that were screened, one had Z' 0.35 which was lower than expected and due to a larger than expected variation in the control populations on left/right hand side of plate. The remaining 6 plates had Z' >0.59. All 21 of the hit compounds that were shown to be active previously, see Figure 13(B), showed a similar trend in the n=3 data set, see Figure 16(B).

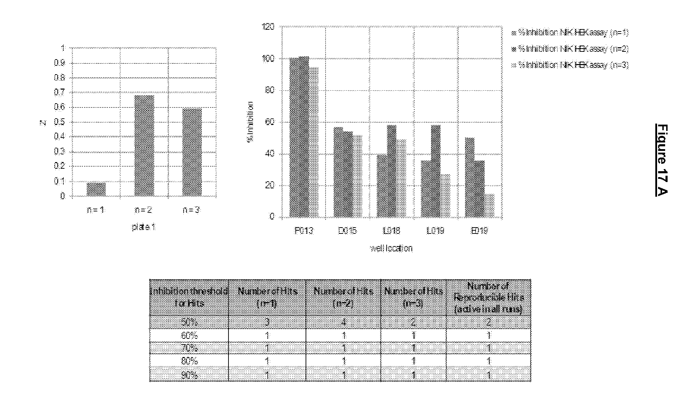

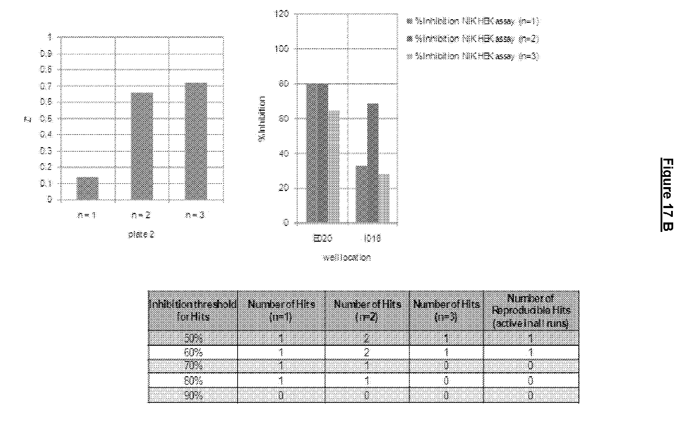

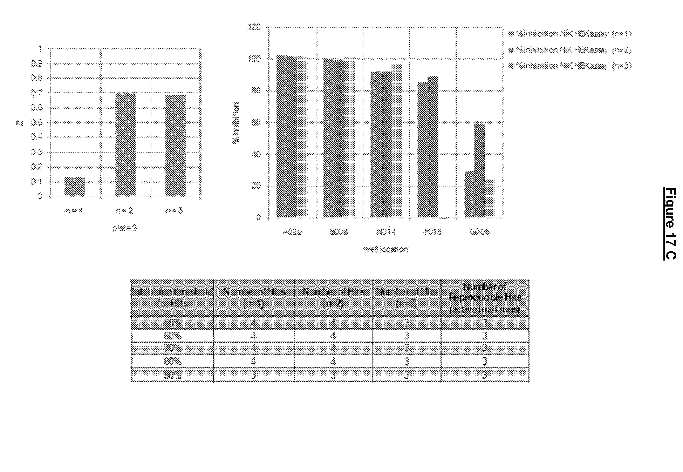

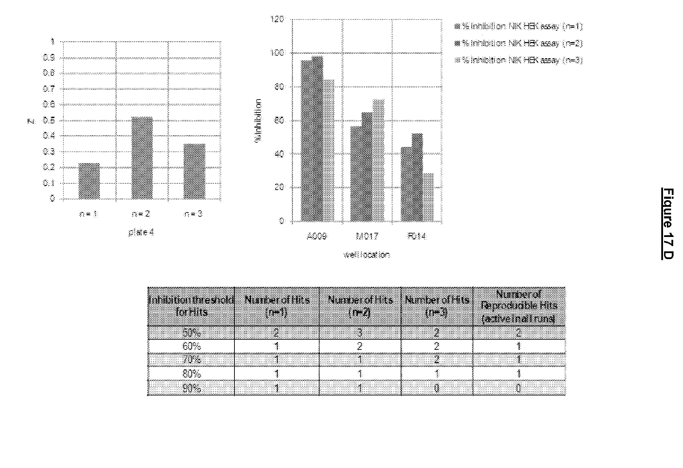

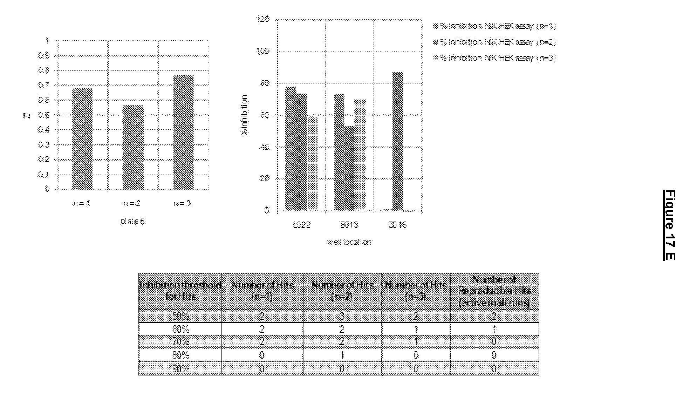

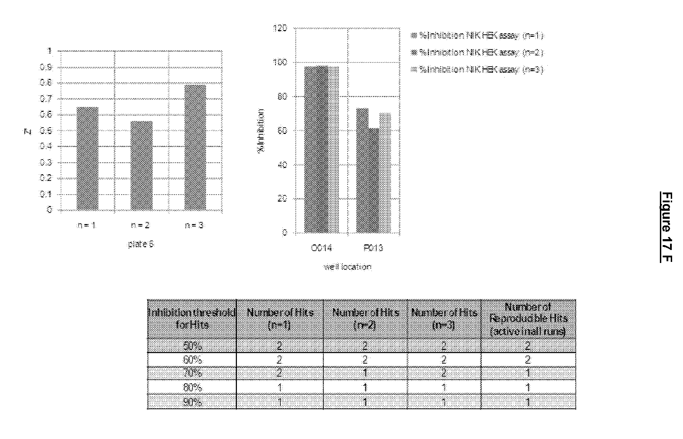

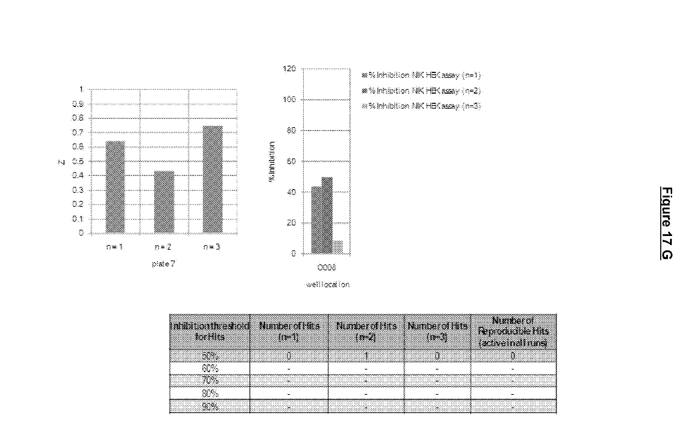

A plate by plate analysis was performed for the triplicate data sets in order to determine the reproducibility of the hits, see Figure 17.

The data in Figure 17 suggests that despite the lower than anticipated Z' for 5 plates, the most active compounds in general are reproducible.

### Reference list

1. Coope H.J., Atkinson P.G., Huhse B., Belich M., Janzen J., Holman M.J., Klaus G.G., Johnston L.H., Ley S.C. CD40 regulates the processing of NF-kappaB2 p100 to p52. EMBO J 2002, 15:5375-5385.
2. Dejardin E., The alternative NF-kappaB pathway from biochemistry to biology: pitfalls and promises for future drug development. Biochem Pharmacol 2006, 72:1161-1179.
3. Garrick R.A. and Chinard F.P. Membrane permeability of isolated lung cells to nonelectrolytes at different temperatures. Am J Physiol 1982, 243:C285-C292
4. Hassan, N.J. Gul S., Flett F., Hollingsworth E., Dunne A.A., Emmons A.J., Hutchinson J.P. et al. Development of an insect-cell-based assay for detection of kinase inhibition using NF-kappaB-inducing kinase as a paradigm. Biochem J 2009, 419:65-73.
5. Hayden M.S. and Ghosh S. Shared principles in NF-kappaB signaling. Cell 2008, 132:344-362.
6. Ling L., Cao Z., Goeddel D.V. NF-kappaB-inducing kinase activates IKK-alpha by phosphorylation of Ser-176. Proc Natl Acad Sci 1998, 95:3792-3797.
7. Malinin N.L., Boldin M.P., Kovalenko A.V., Wallach D. MAP3K-related kinase involved in NF-kappaB induction by TNF, CD95 and IL-1. Nature 1997, 385:540-544.
8. Mortier J., Masereel B., Remouchamps C., Ganeff C., Piette J., Frederick R. NF-kappaB inducing kinase (NIK) inhibitors: identification of new scaffolds using virtual screening. Bioorg & Med Chem Lett 2010, 20:4515-4520.
9. Nakano H., Shindo M., Sakon S., Nishinaka S., Mihara M., Yagita H., Okumura K. Differential regulation of IkappaB kinase alpha and beta by two upstream kinases, NF-kappaB-inducing kinase and mitogen-activated protein kinase/ERK kinase kinase-1. Proc Natl Acad Sci 1998, 95:3537-3542.
10. Qing G., Qu Z., Xiao G. Stabilization of basally translated NF-kappaB-inducing kinase (NIK) protein functions as a molecular switch of processing of NF-kappaB2 p100. J Biol Chem 2005, 280(49):40578-82.
11. Shinkura R., Kitada K., Matsuda F., Tashiro K., Ikuta K., Suzuki M., Kogishi K., Serikawa T., Honjo T. Nature Genetics 1999, 22:74-77.
12. Sun S.C., Non-canonical NF-κB signaling pathway Cell Res 2011, 21:71-85.
13. Thu Y.M. and Richmond A. NF-κB inducing kinase: a key regulator in the immune system and in cancer. Cytokine & Growth Factor Rev 2010, 21:213-226.
14. Vallabhapurapu S. and Karin M. Regulation and function of NF-kappaB transcription factors in the immune system. Annu Rev Immunol 2009, 27:693-733.
15. Xiao G., Harhaj E.W., Sun S.C. NF-kappaB-inducing kinase regulates the processing of NF-kappaB2 p100. Mol Cell 2001, 7:401-409.
16. Xiao G., Fong A., Sun S.C. Induction of p100 processing by NF-kappaB-inducing kinase involves docking IkappaB kinase alpha (IKKalpha) to p100 and IKKalpha-mediated phosphorylation. J Biol Chem 2004, 279:30099-30105.
17. US 6,841,556
18. WO 2009/158011

## Claims

1. A method for identifying a compound that inhibits NIK activity, comprising:
(a) providing HEK cells containing a DNA encoding for NIK and a DNA encoding for IKK1-KD (transfected HEK cells) in a culture medium;
(b) incubating said transfected HEK cells with and without a candidate molecule to be tested at a temperature of 37°C +/- 2°C in the presence of 5% CO₂ for 24 hours;
(c) removing the culture medium;
(d) lysing the cells;
(e) adding to the cell lysate an acceptor complex followed by a donor complex;
(f) exciting the cell lysate with a laser illumination and
(g) measuring the signal arising upon laser excitation of cell lysate using a bead-based luminescent proximity detection system,
wherein a reduction or the absence of the signal obtained from a cell lysate deriving from transfected HEK cells cultured in the presence of a test compound is indicative of the inhibition of NIK-mediated phosphorylation of IKK1-KD, and wherein the acceptor complex comprises a molecule-coated bead and a binding partner which binds to the IKK1-KD protein and the donor complex comprises a molecule-coated bead and a binding partner which binds to the IKK1-KD protein.

2. A method according to claim 1, wherein a reduction of the signal obtained from a cell lysate deriving from transfected HEK cells cultured in the presence of a test compound compared to the signal obtained from a cell lysate deriving from transfected HEK cells cultured in the absence of a test compound is indicative of a reduction of NIK-mediated phosphorylation of IKK1-KD.

3. A method according to claim 1 or 2, wherein a DNA encoding for NIK and a DNA encoding for IKK1-KD are inserted into HEK cells by transfecting said HEK cells with a first expression vector containing a DNA encoding for NIK and a second expression vector containing a DNA encoding for IKK1-KD.

4. A method according to any of the preceding claims, wherein the molecule-coated bead of the acceptor complex is a Protein-A coated bead and the binding partner of the acceptor complex is an antibody which specifically binds to the phosphorylated form of Ser-176 residue of IKK1-KD.

5. A method according to any of the preceding claims, wherein the molecule-coated bead of the donor complex is a streptavidin-coated bead and the binding partner of the donor complex is a biotinylated antibody which binds to IKK1-KD.

6. A method according to any of the preceding claims, wherein the bead-based luminescent proximity detection system is AlphaScreen detection system.

7. A method according to any of the preceding claims, further comprising the step of testing those candidate molecule showing a NIK inhibitory activity in a cytotoxic assay, thus allowing to distinguish candidate molecules having a NIK inhibitor activity associated to a cytotoxic effect from candidate molecules having NIK inhibitor activity not associated to a cytotoxic effect.
